# EUROPÄISCHE PATENTSCHRIFT

(11) **EP 1 691 606 B1**
(45) Veröffentlichungstag und Bekanntmachung des Hinweises auf die Patenterteilung: **04.04.2007**
(21) Anmeldenummer: 04797950.5
(22) Anmeldetag: 17.11.2004
(51) Int. Cl.: A01N 25/26, A01N 25/32, A01N 59/16, A61L 15/18, A61L 15/46, A61L 27/30, A61L 27/54, A61L 29/10, A61L 29/16, A61L 31/08, A61L 31/16, C23C 14/00, C23C 16/00

(54) **ANTIMIKROBIELLES SCHICHTMATERIAL**
ANTIMICROBIAL COMPOSITE MATERIAL
MATIERE DE REVETEMENT ANTIMICROBIENNE

(30) Priorität: 17.11.2003 DE 10353756
(43) Veröffentlichungstag der Anmeldung: 23.08.2006
(60) Teilanmeldung: 07102968.0
(73) Patentinhaber: Bio-Gate AG, 28359 Bremen (DE); Fraunhofer-Gesellschaft zur Förderung der angewandten Forschung e.V., 80686 München (DE)
(72) Erfinder: WAGENER, Michael, 28355 Bremen (DE); VISSING, Klaus Dieter, 27321 Morsum (DE); SALZ, Dirk, 28195 Bremen (DE); STEINRÜCKE, Peter, 91052 Erlangen (DE)
(74) Vertreter: Eisenführ, Speiser & Partner
(86) Internationale Anmeldenummer: PCT/EP2004/013030
(87) Internationale Veröffentlichungsnummer: WO 2005/048708

(56) Entgegenhaltungen:
- EP-A- 0 328 421
- EP-A- 0 570 944
- WO-A1-85/02422
- US-A- 5 595 750
- US-A1- 2003 118 664

## Beschreibung

Die Erfindung betrifft ein antimikrobielles und nicht zytotoxisches Schichtmaterial, entsprechende Medizinprodukte und Herstellungsverfahren.

Es besteht ein unablässiger Bedarf in vielfältigen Anwendungsgebieten, die Ansiedlung, die Vermehrung und das Überleben von Mikroorganismen, insbesondere Prokaryonten und Pilzen, zu steuern. Insbesondere ist es vielfach gewünscht, die Konzentration von Mikroorganismen auf einer bestimmten Fläche zu begrenzen oder diese Fläche gänzlich von Mikroorganismen - gegebenenfalls Mikroorganismen einer bestimmten Art oder Gattung - freizuhalten. Dieses Ziel wird insbesondere in im weitesten Sinne medizinischen, medizintechnischen oder hygienetechnischen Anwendungen angestrebt. Herkömmlicherweise werden deshalb beispielsweise im Bereich von Medizin- und Hygieneprodukten antimikrobiell wirksame Werkstoffe und Beschichtungen verwendet, beispielsweise silberbeschichtete Fäden für die Chirurgie (siehe S. Silver, FEMS Microbiology Reviews (2003): 341 bis 353) oder kupferhaltige Antifoulinglacke. Als besonders wirksam haben sich dabei breitbandig wirksame Biozide und hierbei insbesondere anorganische Biozide wie beispielsweise Silber und dessen Ionen erwiesen. Das mit dem Biozid behandelte Material setzt dabei im Laufe der Zeit das in ihm enthaltene Biozid frei und verringert oder verhindert vollständig die Ansiedlung oder Vermehrung von Mikroorganismen auf dem Material selbst, aber auch in seiner Umgebung.

Dabei ist häufig problematisch, dass die herkömmlichen antimikrobiell wirksamen Materialien anfänglich eine hohe Biozid-Konzentration freisetzen, so dass die Konzentration des freigesetzten Biozids nicht nur auf die zu bekämpfenden Mikroorganismen, sondern ungewollt auch auf höhere Zellen toxisch wirkt. Dies ist insbesondere bei Medizinprodukten wie Wundauflagen, Kathetern, Kontaktlinsen und Implantaten störend, da ein so behandeltes Medizinprodukt die Wundheilung verzögern und Gewebereizungen und Allergien hervorrufen kann. Entsprechende Nachteile treten auch bei Biozid-freisetzenden Hygieneprodukten wie beispielsweise Binden, Tampons oder Windeln sowie bei der Herstellung und Verarbeitung von Lebensmitteln auf, insbesondere im Zusammenhang mit Biozid-freisetzenden Verpackungen sowie Biozid-freisetzenden Bauteilen zum Herstellen oder Verarbeiten von Lebensmitteln. Darüber hinaus wird die antimikrobielle Wirkung durch Auslaugung des mit dem bioziden Wirkstoff versehenen Materials rasch erschöpft. Im übrigen ist bei herkömmlichen Beschichtungen nachteilig, dass diese bei einer Beschädigung beispielsweise durch Abrieb, wie sie auch bei bestimmungsgemäßem Gebrauch der entsprechend beschichteten Gegenstände auftreten kann, oft zumindest lokal eine sehr hohe Menge Biozid freisetzen können.

Zum Beheben dieser Nachteile wird gemäß der WO 03/024494 ein antimikrobieller Kleb- und Beschichtungsstoff vorgeschlagen, der metallische Silber-Partikel mit einem Gehalt von weniger als 5 ppm an Silber-, Natrium- und Kalium-Ionen enthält, wobei es sich bei dem Kleb- und Beschichtungsstoff um ein synthetisch hergestelltes Material auf organischer Basis handelt, das im Allgemeinen nach der Verarbeitung aushärtet. Die Silber-Partikel sind dabei gleichmäßig im Kleb- und Beschichtungsstoff verteilt. Insbesondere soll der Kleb- und Beschichtungsstoff ein Lack oder Klebstoff insbesondere auf duro- oder thermoplastischer Basis sein. Nachteilig hieran ist jedoch, dass die Metallionenfreisetzungsrate nur schwer steuer- oder einstellbar ist.

Aus der US 2002/0006887 A1 ist ein mit einem antimikrobiellen Wirkstoff versehener Wischer bekannt. Zum Verzögern der Abgabe des antimikrobiellen Wirkstoffs ist der Wischer mit einer Polyethylen-Vinylacetatschicht versehen, wobei der antimikrobielle Wirkstoff Calciumhypochlorit in der Beschichtung ganz oder teilweise verkapselt ist. Die freigesetzten Wirkstoffmengen sind dabei jedoch viel zu hoch, um eine zytotoxische Wirkung ausschließen zu können. Die Anwendung einer Beschichtung wie in diesem Dokument beschrieben ist daher nur für einen Wischer sinnvoll, nicht jedoch allgemein für medizinische Produkte, und insbesondere nicht für implantierbare Produkte.

Aus der WO 00/60297 ist ein mit einem antimikrobiellen Wirkstoff versehenes Rohr bekannt. Dieses enthält jedoch ebenfalls keine Transprotkontrollschicht, die frei von Partikeln des antimikrobiellen Wirkstoffs ist. Der Wirkstoff kommt somit immer unmittelbar in Kontakt mit der Oberfläche des Rohres und kann somit auch in zytotoxischen Konzentrationen abgegeben werden.

Die US 5,595,750 beschreibt anorganische Partikel, die mit einem Biozid beschichtet und dann mit einer Schutzschicht aus z. B. Silika versehen werden. Die Schutzschicht dient als Barriere zwischen dem Biozid und einem Polymer in das die Partikel eingearbeitet werden können. Die Biozid-Schicht und die Schutzschicht werden in wässriger Lösung aufgetragen.

Die WO 85/02422 offenbart antimikrobiell wirksame, geformte Gegenstände mit einer Faser, die Jod als Biozid enthält. Eine poröse, Jod-einfangende Schutzschicht ist zwischen den jodtragenden Fasern und der zum Anwendungsort hin orientierten Oberfläche vorgesehen.

Es war deshalb eine Aufgabe der vorliegenden Erfindung, ein möglichst einfach und kostengünstig herstellbares Schichtmaterial anzugeben, das antimikrobielle Eigenschaften besitzt, jedoch nicht zytotoxisch sein sollte. Dabei ist ein Schichtmaterial antimikrobiell, wenn es die Vermehrung von *Staphylococcus epidermidis* zumindest zehn Stunden lang hemmt, gemessen wie beschrieben in DE 197 58 598 A1. Dabei wird bestimmt, ob Bakterien beispielsweise der vorgenannten Art auf der Oberfläche des Schichtmaterials im Vergleich zu einer biozidfreien Kontrollprobe nur noch weniger als 0,1% and Tochterzellen innerhalb von 18 h produzieren können. Ein Schichtmaterial ist ferner zytotoxisch, wenn es eine zytotoxische Wirkung wie in DIN-ISO 10993-5 beschrieben aufweist. Das Schichtmaterial sollte zudem eine möglichst langanhaltende antimikrobielle und nicht zytotoxische Wirkung besitzen. Es sollte möglichst universell einsetzbar sein, beispielsweise auf Vliesstoffen, Folien, Kunststoffen, Metallen und Materialkombinationen, und die Herstellung auch dünner Beschichtungen insbesondere von 40 bis 200 nm Dicke ermöglichen. Das Schichtmaterial sollte zudem möglichst wenig Biozid enthalten. Die Oberflächeneigenschaften des Schichtmaterials sollten über einen möglichst weiten Bereich einstellbar sein. Das Schichtmaterial sollte zudem eine möglichst gute Haftung auf einem zu beschichtenden Substrat besitzen, es sollte möglichst transparent, lebensmittelbeständig und hydrolysestabil sein und einstellbare Sperrschichteigenschaften besitzen.

Erfindungsgemäß wird deshalb ein antimikrobielles und nicht zytotoxisches Schichtmaterial vorgeschlagen, umfassend
a) eine Biozid-Schicht mit einem bioziden Wirkstoff und
b) eine die Biozid-Schicht bedeckende Transportkontrollschicht mit einer Dicke und einer Porosität, die eingestellt sind, um den bioziden Wirkstoff aus der Biozid-Schicht durch die Transportkontrollschicht hindurch in einer antimikrobiellen und nicht zytotoxischen Menge abzugeben, wobei die Transportkontrollschicht eine plasmapolymere Schicht und/oder aufgesputterte Schicht ist, und wobei die Transportkontrollschicht einen Silizium-Anteil von 20-60 %, einen Kohlenstoffanteil von 10-30 % und einen Sauerstoffanteil von 30-50 % besitzt.

Gegenüber herkömmlichen antimikrobiellen Materialien ermöglicht es das erfindungsgemäße Schichtmaterial, gleichzeitig eine hohe Biozid-Konzentration im Schichtmaterial selbst vorzusehen, die normalerweise zytotoxisch wirken würde, wobei die Gesamtmenge an Biozid vorteilhaft gering bleiben kann. Die Biozid-Schicht bildet gleichsam ein Depot des bioziden Wirkstoffs, um eine langanhaltende Abgabe des bioziden Wirkstoffs zu ermöglichen. Durch Vorsehen der Transportkontrollschicht die Konzentration des aus der Biozid-Schicht durch die Transportkontrollschicht hindurch abgegebenen bioziden Wirkstoffs zu begrenzen, so dass dieser nicht mehr zytotoxisch, gleichzeitig jedoch auch noch immer antimikrobiell wirkt. Die Transportkontrollschicht kann somit eine steuernde und regulierende Funktion besitzen. Darüber hinaus kann die Transportkontrollschicht einen direkten Kontakt der Umgebung mit der Biozid-Schicht verhindern. Dadurch wird die Haltbarkeit des Schichtmaterials verbessert, da beispielsweise der korrosive Angriff von Körperflüssigkeiten oder Lebensmitteln (insbesondere Säfte) wirkungsvoll unterbunden oder eingeschränkt werden kann. Die Transportkontrollschicht kann auf beiden Seiten oder nur auf einer Seite der Biozid-Schicht angeordnet sein. Letzteres ist insbesondere dann bevorzugt, wenn das erfindungsgemäße Schichtmaterial eine Beschichtung auf einem festen Körper bildet. In einem solchen Fall kann der mit dem erfindungsgemäßen Schichtmaterial beschichtete Körper die nicht von der Transportkontrollschicht bedeckte Seite der Biozid-Schicht bedecken.

Im Sinne der vorliegenden Erfindung ist ein biozider Wirkstoff jede Substanz, die eine antimikrobielle Wirkung im oben beschriebenen Sinne entfalten kann (biozider Wirkstoff im engeren Sinne). Zu den bioziden Wirkstoffen werden auch solche Stoffe gezählt, die durch Umwandlung den bioziden Wirkstoff im engeren Sinne in der Umgebung hervorbringen, in der ein jeweiliges Schichtmaterial bestimmungsgemäß verwendet werden soll. Ist beispielsweise der biozide Wirkstoff im engeren Sinne ein Metallion, insbesondere ein Silber-, Kupfer- und/oder Zink-Kation, so sind auch metallisches Silber, Kupfer bzw. Zink und Legierungen, Komplexe und andere Substanzen biozide Wirkstoffe, aus denen die genannten Kationen in einem geeigneten Umfeld freigesetzt werden können, beispielsweise im Bereich einer Wunde. Metallische Biozide sind erfindungsgemäß bevorzugt.

Der Fachmann versteht, dass ein erfindungsgemäßes Schichtmaterial auch gegen andere Mikroorganismen und nicht oder nicht nur gegen *Staphylococcus epidemidis* antimikrobiell wirksam sein kann. Die antimikrobielle Wirksamkeit des erfindungsgemäßen Schichtmaterials im Hinblick auf andere Mikroorganismen wird entsprechend der DE 197 58 598 A1 mit dem jeweils zu untersuchenden Mikroorganismus anstelle von *Staphylococcus epidermidis* durchgeführt. Besonders bevorzugt sind solche erfindungsgemäßen Schichtmaterialien, die, ohne zytotoxisch zu sein, antimikrobiell wirksam sind gegen einen oder mehrere der Mikroorganismen der Gruppe *Bacillus, Clostridium, Enterobacter, Escherichia, Pseudomonas, Salmonella, Staphylococcus, Yersinia, Candida, Listeria.* Das erfindungsgemäße Schichtmaterial kann zudem eine antivirale Wirkung besitzen.

Die Transportkontrollschicht des erfindungsgemäßen Schichtmaterials ist vorzugsweise so ausgebildet, dass sie eine Gasdurchlässigkeit für Sauerstoff (O₂) im Bereich von 100 bis 1000 (cm³ bar) / (Tag m²) aufweist, vorzugsweise 600 bis 700 (cm³ bar) / (Tag m²). Solche Transportkontrollschichten können besonders zweckmäßig durch Vakuumaufdampfprozesse oder Plasmapolymerisation erzeugt werden. Anhand des soeben beschriebenen Gasdurchlässigkeitskriteriums kann der Fachmann unter Durchführung üblicher Routineversuche geeignete Ausgangsmaterialien und Parameter für die Herstellung einer entsprechenden Transportkontrollschicht ermitteln. Besonders bevorzugte Transportkontrollschichten sind im weiteren Verlauf dieser Beschreibung und in den Beispielen angegeben.

Durch die oben genannten vakuumgestützten Dünnschichtverfahren, insbesondere Sputtern und Plasmapolymerisation, können auf vorteilhaft einfache Weise die Adhäsionseigenschaften, Hydrolysestabilität und Oberflächenenergie der erfindungsgemäßen Schichtmaterialien leicht eingestellt werden. Außerdem sind derart hergestellte Transportkontrollschichten transparent und können auf weitgehend beliebigen Substraten aufgetragen werden. Dabei bestehen keine praktisch relevanten Einschränkungen hinsichtlich der Verarbeitungstemperatur.

Bei vakuumgestützte Dünnschichtverfahren, insbesondere Sputtern und Plasmapolymerisation, wird die Hydrophilie des Schichtmaterials vorzugsweise dadurch beeinflusst, dass der Sauerstoffgehalt der für das Herstellen der Beschichtung gewählten Atmosphäre hoch gewählt wird. Bei einem O₂-Anteil von bis zu 95%, 0,07 mbar Arbeitsatmosphärendruck (Restlicher Anteil der Arbeitsatmosphäre: HMDSO), Plasmaleistung 2500 W, Reaktorvolumen 400 l, wird ein vorteilhaft stark hydrophobes Schichtmaterial erreicht (s. Beispiele). Erfindungsgemäße hydrophile Schichtmaterialien können bevorzugt erhalten werden bei einem O₂-Anteil von 40 bis 95% (Rest der Arbeitsatmosphäre: HMDSO), wobei ein Schichtmaterial im allgemeinen um so hydrophober sein wird, je höher der O₂-Anteil der Arbeitsatmosphäre gewählt wird. So kann auf vorteilhaft einfache Weise auch die Diffusionsrate von Silberionen aus der Transportkontrollschicht beeinflusst werden; die Diffusionsrate ist im allgemeinen höher, je hydrophiler das Schichtmaterial ist.

Ergänzend oder alternativ dazu kann ein hydrophiles Schichtmaterial erfindungsgemäß durch ein vakuumgestütztes Dünnschichtverfahren hergestellt werden, bei dem nach dem Herstellen einer Transportkontrollschicht das Schichtmaterial in einer reinen Sauerstoffatmosphäre bei einem Arbeitsatmosphärendruck von 0,02 bis 0,04 mbar, vorzugsweise 0,06 mbar, und einer Plasmaleistung von 500 bis 2000 W, vorzugsweise 1000 W, in einem 400 I-Reaktor behandelt wird.

Hydrophobe Schichtmaterialien können erfindungsgemäß hergestellt werden durch ein vakuumgestütztes Dünnschichtverfahren mit einer Arbeitsatmosphäre aus Wasserstoff und Hexafluorethan (C₂F₆), gegebenenfalls mit einem Nachaktivierungsschritt. Das Verhältnis von Wasserstoff zu Hexafluorethan beträgt 2:1 bis 4:1, vorzugsweise 3:1, bei einer Plasmaleistung von 400 W und einem Reaktorvolumen von 400 I.

Der Fachmann kann die obigen Angaben leicht an andere Plasmaleistungen und andere Reaktorvolumina anpassen, sollte dies gewünscht sein.

Durch die oben beschriebenen Herstellverfahren wird auch die Oberflächenenergie des erfindungsgemäßen Schichtmaterials beeinflusst, insbesondere kann die Oberflächenenergie frei gewählt werden im Bereich von 10 bis 105 mN/m. Je höher die Oberflächenenergie, desto geringer ist die Adhäsionsneigung von Staphylococcus epidermidis und anderen Mikroorganismen. Durch das erfindungsgemäße Einstellen der Oberflächenenergie wird daher die antimikrobielle Wirkung der erfindungsgemäßen Schichtmaterialien vorteilhaft einfach steuerbar. Ferner wird es möglich, zielgerichtet die Adhäsion Gram-positiver und/oder Gramnegativer Mikroorganismen einzustellen. Zusätzlich wird eine hohe Biokompatibilität durch eine silikatähnliche Struktur erreichbar.

Besonderes bevorzugt sind solche erfindungsgemäßen Schichtmaterialien, bei denen der biozide Wirkstoff ein anorganisches Biozid ist. Solche bioziden Wirkstoffe sind gewöhnlich preiswert, leicht erhältlich und leicht zu verarbeiten. Der biozide Wirkstoff kann durch verschiedene Verfahren vorgelegt werden, insbesondere kann er auf einer Oberfläche aufgebracht werden, die mit einem erfindungsgemäßen Schichtmaterial beschichtet werden soll. Zum Aufbringen eines anorganischen bioziden Wirkstoffs besonders geeignet sind Vakuumverdampfen, Sputtern und chemical vapor deposition.

In einer besonders bevorzugten Ausführungsform des erfindungsgemäßen Schichtmaterials ist der biozide Wirkstoff ausgewählt aus der Gruppe bestehend aus Silber, Kupfer und Zink, deren Ionen und deren Metallkomplexe oder eine Mischung oder Legierung dieser Elemente. Diese bioziden Wirkstoffe wirken gegen eine Vielzahl verschiedener Mikroorganismen und greifen auf zahlreiche Weisen in deren Stoffwechsel ein. Dementsprechend kommt es bei Verwendung dieser bioziden Wirkstoffe seltener zur Resistenzbildung bei Bakterien als bei Verwendung spezifisch wirkender organischer Biozide, insbesondere Antibiotika.

Als besonders vorteilhaft hat sich dabei ein solches erfindungsgemäßes Schichtmaterial herausgestellt, bei dem der biozide Wirkstoff Silber, ein SilberKation oder ein Silber- bzw. Silberkation-freisetzender Komplex oder eine solche Legierung ist. Insbesondere metallisches Silber ist leicht verarbeitbar und in hoher Qualität zu einem verhältnismäßig geringem Preis erhältlich, so dass auch das erfindungsgemäße Schichtmaterial wiederum verhältnismäßig preiswert hergestellt werden kann.

Zweckmäßigerweise liegt der biozide Wirkstoff im erfindungsgemäßen Schichtmaterial in körniger Form vor, wobei eine mittlere Korngröße der Primärpartikel von 5 bis 100 nm bevorzugt ist. Solche feinen Pulver biozider Wirkstoffe lassen sich insbesondere für anorganische Biozide, und hierbei insbesondere für Silber, aber auch für Kupfer und Zink, sowie Mischungen, Komplexe und Legierungen der drei genannten Metalle leicht herstellen. Aufgrund der geringen mittleren Korngröße besitzt der biozide Wirkstoff eine hohe spezifische Oberfläche, so dass er insbesondere durch Diffusion gut aus der Biozid-Schicht heraus abgegeben werden kann. Ferner ist vorteilhaft, dass aufgrund der hohen spezifischen Oberfläche eine chemische Inaktivierung des körnigen Wirkstoffs, wie sie beispielsweise in Wundumgebungen gelegentlich zu besorgen ist, gewöhnlich nur einen Teil der Oberfläche betrifft, so dass eine Abgabe des bioziden Wirkstoff aus der Biozid-Schicht heraus auch unter widrigen Bedingen ermöglicht wird. Als besonders vorteilhaft haben sich solche erfindungsgemäßen Schichtmaterialien erwiesen, bei denen die mittlere Korngröße des bioziden Wirkstoffs 5 bis 50 nm, vorzugsweise 5 bis 20 nm. Wenn der biozide Wirkstoff Silber oder eine Silber-Legierung ist, so spricht man bei diesen Korngrößenverteilungen auch von nanoskaligem (nano scale) Silber bzw. einer nanoskaligen Silber-Legierung.

Die Biozid-Schicht kann, je nach Anwendungsbereich, eine Dicke von zumindest 1 nm, und vorzugsweise nicht mehr als 1 mm besitzen. Bei Verwendung körniger biozider Wirkstoffe ist die Biozid-Schicht zumindest so dick wie der körnige Wirkstoff. Vorzugsweise beträgt die Dicke der Biozid-Schicht zumindest 5 nm bis 100 nm, wobei besonders Schichtdicken von 10 nm bis 50 nm bevorzugt werden, insbesondere wenn der biozide Wirkstoff Silber, Kupfer und/oder Zink bzw. deren Ionen, Metallkomplexe oder eine Mischung oder Legierung dieser Elemente ist. Es hat sich gezeigt, dass in einem erfindungsgemäßen Schichtmaterial bereits derartig geringe Schichtdicken eines bioziden Wirkstoffs (insbesondere eines bioziden Wirkstoffs enthaltend nanoskaliges Silber) ausreichend sind, um eine antimikrobielle, nicht zytotoxische Wirkung dauerhaft erreichen zu können.

Dabei wird die Biozid-Schicht vorzugsweise nicht vollflächig auf das mit dem Schichtmaterial versehene Substrat aufgebracht, sondern bedeckt nur einen Teil dieses Substrats. Die Transportkontrollschicht steht dann örtlich begrenzt unmittelbar in Kontakt mit dem Substrat und haftet daher besonders gut auf dem Substrat. Dieser verbesserte Haftung der Transportkontrollschicht verbessert zudem die Haftung eines körnigen bioziden Wirkstoffs wie beispielsweise Silberpartikel, insbesondere nanoskaliges Silber.

Zur Herstellung des erfindungsgemäßen Schichtmaterials eignen sich vakuumgestützte Verfahren sehr gut, insbesondere wenn die Herstellung sehr dünner Schichten erforderlich ist. Die Biozid-Schicht wird dann besonders bevorzugt über einen Sputter- oder einen Aufdampfprozess hergestellt, da hier metallische Biozide direkt auf dem Substrat abgeschieden werden können, ohne dass ein chemischer Prozess abläuft. Im Vergleich hierzu wird bei Imprägnierungs- oder Sol-Gel-Methoden ein Metallsalz verwendet, dass im oder auf dem Substrat zum Metall reduziert wird. Es ist gerade dieser Reduktionsprozess, der häufig nicht vollständig abläuft und somit die Herstellung schwer reproduzierbar macht. Zudem entstehen bei der Herstellung herkömmlicher Beschichtungen, insbesondere mit Sol-Gel-Methoden, Rückstände, die aufwendig abgewaschen und entsorgt werden müssen. Derartige Rückstände können mit erfindungsgemäß durch vakuumgestützte Dünnschichtverfahren hergestellten Schichtmaterialien vermieden werden.

Ferner ist ein erfindungsgemäßes Schichtmaterial bevorzugt, bei der die Biozid-Schicht ferner umfasst: Gold, Platin, Palladium, Iridium, Zinn, Antimon, deren Ionen, deren Metallkomplexe, oder eine Mischung oder Legierung des bioziden Wirkstoffs mit einem oder mehreren dieser Elemente. Der Zusatz der genannten Elemente zum bioziden Wirkstoff erhöht und/oder verlängert die antimikrobielle Wirksamkeit. Die genannten Elemente liegen vorzugsweise in kationischer Form gebunden in Ionenaustauschern, in Form eines Komplexes oder als Salz, vorzugsweise einer polymeren Carbonsäure, vor.

Darüber hinaus ist ein erfindungsgemäßes Schichtmaterial bevorzugt, bei dem die Transportkontrollschicht ein Grundmaterial besitzt, das ausgewählt ist aus der Gruppe bestehend aus
a) einem organischen Grundmaterial, insbesondere einem Plasmapolymer, einem Sol-Gel, einem Lack, und einem silikonisierten Grundmaterial, oder
b) einem anorganischen Grundmaterial, insbesondere SiO₂ und SiC, einem Metalloxid, insbesondere TiO₂ und Al₂O₃, und einem nicht-bioziden Metall, insbesondere Titan oder medizinischem Edelstahl.

Es versteht sich dabei, dass das Grundmaterial eine Dicke und Porosität besitzt, um eine Abgabe des bioziden Wirkstoffs durch die Transportkontrollschicht hindurch in einer Konzentration zu ermöglichen, bei der der so abgegebene biozide Wirkstoff antimikrobiell und nicht zytotoxisch wirken kann. Hierbei ist besonders bevorzugt, wenn das Grundmaterial mikroporös ist. Insbesondere zum Herstellen dünner Schichten ist es bevorzugt, die Transportkontrollschicht durch Plasma-Polymerisationsverfahren oder durch Aufsputtern herzustellen. Auf diese Weise können sehr dünne Transportkontrollschichten hergestellt werden, durch die biozide Wirkstoffe, wie beispielsweise atomares oder kationisches Silber diffundieren und dem Schichtmaterial seine antimikrobielle, nicht zytotoxische Wirkung verleihen können.

Die Transportkontrollschicht wird vorzugsweise so hergestellt, dass ihre Schichtdicke, Dichte, ihr Feuchtigkeitsaufnahmevermögen, ihre Diffusionsdichtigkeit gegen Wasserdampf, ihre chemische Zusammensetzung und ihre Vernetzungsstruktur eine Abgabe des bioziden Wirkstoffs durch die Transportkontrollschicht hindurch ermöglicht, so dass der so abgegebene biozide Wirkstoff antimikrobiell und nicht zytotoxisch wirken kann. Dient beispielsweise eine gesputterte oder plasmapolymere Schicht als Transportkontrollschicht, so ist diese vorzugsweise stark vernetzt und besitzt eine hohe Diffusionsdichtigkeit gegen Wasserdampf und andere Gase oder Dämpfe sowie ein geringes Feuchtigkeitsaufnahmevermögen. Eine derartige Transportkontrollschicht benötigt nur eine sehr geringe Schichtdicke, um noch eine ausreichende antimikrobielle, aber noch nicht zytotoxische Wirksamkeit des bioziden Wirkstoffs zu gewährleisten.

Die Transportkontrollschicht ist vorzugsweise so gewählt, dass eine möglichst niedrige Bakterienadhäsion stattfindet. Dies kann z.B. durch die Einstellung der Oberflächenenergie in Abhängigkeit der untersuchten Bakterienart erreicht werden. Die Oberflächenenergie wird über die Schichtabscheidungsparameter wie in Beispiel 7 angegeben eingestellt. Die quantitative Messung der Bakterienadhäsion wird mittels der in DE 197 51 581 C2 beschriebenen Methode durchgeführt. Dadurch können die Schichteigenschaften in Bezug auf Biokompatibilität (insbesondere nicht zytotoxische Eigenschaften) bei möglichst niedrigem Biozidanteil optimiert werden.

Die erfindungsgemäße Transportkontrollschicht ermöglicht daher, sowohl die Zytotoxizität als auch Oberflächeneigenschaften wie Bakterienadhäsion und Adhäsion von Biomolekülen und Zellen eines vorgewählten Gewebetyps zielgerichtet zu fördern oder zu unterdrücken.

Bei einem erfindungsgemäßen Schichtmaterial besitzt die Transportkontrollschicht einen Siliciumanteil von 20 bis 60 %, vorzugsweise von 20 bis 33 %, einen Kohlenstoffanteil von bis zu 50%, insbesondere von 10 bis 30 %, und einen Sauerstoffanteil von 25 bis 66%, insbesondere auch von 30 bis 50 %. Es versteht sich dabei, dass die Anteile so aufeinander abgestimmt sein müssen, dass sie insgesamt nicht mehr als 100 % ergeben. Die Anteile werden dabei durch X-ray Photoelectron-Spektroskopie (XPS) ermittelt; dabei bleiben Elemente bei der Bestimmung des Silicium-, Kohlenstoff- und Sauerstoffanteils außer Betracht, die beispielsweise wie Wasserstoff nicht durch XPS-Analyse bestimmt werden können. Es können also neben Silicium, Kohlenstoff und Sauerstoff noch weitere Elemente in der Transportkontrollschicht vorhanden sein (nämlich solche, die durch XPS nicht nachgewiesen werden können), ohne dass diese weiteren Elemente bei der Bestimmung des Silicium-, Kohlenstoff- und Sauerstoffanteils berücksichtigt würden. Der Silicium-, Kohlenstoff und Sauerstoffanteil wird in Atomprozent bzw. Molprozent der durch XPS-Analyse nachweisbaren Elemente angegeben.

Die Transportkontrollschicht eines erfindungsgemäßen Schichtmaterials hat vorzugsweise eine mittlere Dicke von 5 nm bis 500 nm. Insbesondere bei Verwendung einer plasmapolymeren Transportkontrollschicht ist es jedoch bevorzugt, wenn die Transportkontrollschicht eine Dicke von 5 bis 200 nm, besonders bevorzugt jedoch nicht mehr als 100 nm, vorzugsweise 10 bis 100 nm besitzt. Bei diesen Schichtdicken lassen sich insbesondere mit durch Plasmapolymerisation hergestellte Transportkontrollschichten hervorragende antimikrobielle und nicht zytotoxische Schichtmaterialien herstellen. Gleichzeitig sind diese Transportkontrollschichten sehr dünn, so dass sie optisch kaum auffallen oder sogar transparent sein können.

Erfindungsgemäß umfasst das Schichtmaterial eine durch Sputtern oder Plasmapolymerisation herstellbaren Transportkontrollschicht. Bei dieser Herstellungsart kann eine besonders gute Beschichtung auch komplex geformter Körper erreicht werden, insbesondere können feinporige Körper, insbesondere Vliesstoffe, sicher mit einer Transportkontrollschicht versehen werden, wobei deren Beweglichkeit, Durchlässigkeit und Atmungsaktivität erhalten bleiben. Zudem ermöglicht das Sputtern und die Plasmapolymerisation die Beschichtung von Substraten, die in Dickschichtverfahren nur mit erheblichen Nachteilen zu beschichten sind; hierzu gehören insbesondere Knochennägel und andere Knochenimplantate. Bei diesen Substraten kann es bei einer herkömmlichen Beschichtung insbesondere passieren, dass die Beschichtung bei der weiteren Verarbeitung des Substrats, insbesondere beim Einbau in einen Knochen, abgeschoben wird und einen lokalen Wulst bildet; in diesem Fall wäre die Freisetzungsrate des bioziden Wirkstoffs nicht mehr über den gesamten Substratkörper einheitlich und steuerbar. Insbesondere könnte es zur Freisetzung des bioziden Wirkstoffs in einer zytotoxischen Konzentration kommen, wodurch insbesondere Heilungsprozesse verzögert oder verhindert werden könnten. Über Plasmapolymerisation sind mit den erfindungsgemäßen Schichtmaterialien auch Transportkontroll-Gradientschichten herstellbar, deren Oberflächeneigenschaften (insbesondere hydrophil, hydrophob, antihaftend und transparent, dazu unten mehr) in vorgewählter Weise von Ort zu Ort unterschiedlich sein können. Zudem kann der Schichtaufbau beim Sputterri oder während der Plasmapolymerisation zum Beispiel ellipsometrisch während der Abscheidung verfolgt werden, um die Reproduzierbarkeit des Schichtaufbaus zu sichern. Dieselbe Kontrolle kann auch während der Abscheidung des Biozids mit einem Sputter- oder Aufdampfverfahren erfolgen.

Ferner sind dünne (vorzugsweise bis zu 100 nm, s.o.) erfindungsgemäße Schichtmaterialien bevorzugt. Diese Schichtmaterialien besitzen vorteilhafte Siegeleigenschaften, wodurch ihre Verwendung als Beschichtung für Verpackungen im Lebensmittel- und Medizinalbereich möglich wird.

Besonders vorteilhaft ist es, wenn die Biozid-Schicht und Transportkontrollschicht beide gemeinsame Grundmaterialien besitzen. Auf diese Weise ist es insbesondere möglich, zunächst einen bioziden Wirkstoff (insbesondere Silber, Kupfer und/oder Zink) in vorzugsweise nanoskaliger Form vorzulegen und anschließend durch Auftragen des Grundmaterials der Transportkontrollschicht in einem einzigen weiteren Arbeitsschritt das erfindungsgemäße Schichtmaterial herzustellen und dabei den bioziden Wirkstoff in diesem Schichtmaterial einzubetten.

Das Grundmaterial der Transportkontrollschicht kann ferner so ausgewählt werden, dass die Transportkontrollschicht außer der Eigenschaft, die Abgabe des bioziden Wirkstoffs durch die Transportkontrollschicht hindurch zu ermöglichen, weitere und vorteilhafte Eigenschaften besitzt. Insbesondere kann die Transportkontrollschicht durch geeignete Wahl des Grundmaterials oder durch weitere Maßnahmen transparent, hydrophil, hydrophob, oleophob und/oder (auch für Bakterien) nicht-haftend sein. Besonders bevorzugt sind hydrophile Transportkontrollschichten für die unten näher beschriebenen medizinischen Produkte wie beispielsweise Wundauflagen. Mit dem erfindungsgemäßen Schichtmaterial versehene medizinische Produkte sind besonders geeignet für eine feuchte Wundbehandlung und für verbessertes Knochenwachstum, indem sie insbesondere Infektionen vorbeugen, ohne Gewebe lokal durch zu hohe Freisetzung des antimikrobiellen Wirkstoffs zu schädigen oder in seiner Heilungsgeschwindigkeit zu hemmen oder diese zu verlangsamen. Erfindungsgemäße Schichtmaterialen mit einer hydrophoberen Transportkontrollschicht wiederum sind besonders bevorzugt dort, wo es auf eine gute Abwischbarkeit und Reinigbarkeit einer Oberfläche ankommt, insbesondere in der Verarbeitung von Lebensmitteln.

Die Biozid-Schicht und auch das erfindungsgemäße Schichtmaterial insgesamt können in beliebiger Form vorliegen. Insbesondere kann die Biozid-Schicht und das erfindungsgemäße Schichtmaterial eine Beschichtung auf einem festen Körper bilden, beispielsweise auf einer Faser, auf einer Metall-, Kunststoff- und/oder Glas-Oberfläche. Die Biozid-Schicht und das erfindungsgemäße Schichtmaterial können aber auch eine Beschichtung auf Partikeln bilden.

Bei Verwendung von Silber (insbesondere nanoskaligem Silber) als bioziden Wirkstoff beträgt der Silbergehalt des erfindungsgemäßen Schichtmaterials vorzugsweise 1 bis 100 ppm. Es sich überraschenderweise gezeigt, dass in einem erfindungsgemäßen Schichtmaterial festes Silber bereits in den angegebenen Mengen eine ausreichend antimikrobielle Wirkung entfalten kann.

Erfindungsgemäß ist das zuvor beschriebene Schichtmaterial einschließlich seiner Ausführungsformen verwendbar zum Herstellen einer antimikrobiellen und nicht zytotoxischen Beschichtung auf einem festen Körper. Insbesondere ist es verwendbar zum Herstellen einer antimikrobiellen und nicht zytotoxischen Beschichtung auf einem Medizinprodukt, insbesondere einem Katheter, einer Wundauflage, einer Kontaktlinse, einem Implantat, einem medizinischen Nagel und/oder Schraube, Knochenfixationsnagel, einem Dentalimplantat, einem medizinischen Instrument, oder auf einem Hygieneprodukt, insbesondere einer Binde, einem Tampon oder einer Windel, oder auf einer Verpackung eines Medizin- oder Hygieneproduktes, oder auf einem Bauteil zum Herstellen oder Verarbeiten von Lebensmitteln oder auf einem sonstigen Produkt, bei dem es eine besondere Hygiene erforderlich ist. Wie eingangs geschildert besteht insbesondere im Bereich der Medizin- und Hygieneprodukte ein Bedarf an antimikrobiellen und gleichzeitig nicht zytotoxischen Produkten. Durch Versehen herkömmlicher Produkte mit einem erfindungsgemäßen Schichtmaterial - beispielsweise in Form einer Beschichtung mit dem Schichtmaterial - kann dieser Bedarf besonders einfach gestillt werden. Die antimikrobielle, nicht-zytotoxische und in ihrer Oberflächenenergie einstellbare Beschichtung eignet sich in besonderer Weise auch zum Beschichten von Dentalimplantaten, wobei durch die Variation der Oberflächenenergie das Einwachsverhalten (Osteointegration) des Implantates verbessert und vorteilhaft einfach eingestellt werden kann.

Die Erfindung wird nachfolgend anhand bevorzugter Ausführungsformen näher beschrieben. Dabei zeigen:
Figur 1: Einen Querschnitt eines antimikrobiellen und nicht zytotoxischen Schichtmaterials,
Figur 2: Einen zeitlichen Verlauf des bakteriellen Bewuchses verschiedener Polyuretan-Oberflächen.

### Beispiel 1: Herstellen eines erfindungsgemäßen Schichtmaterials

Ein festes Substrat, das mit einem erfindungsgemäßen antimikrobiellen und nicht zytotoxischem Schichtmaterial versehen werden soll, wird in einem ersten Beschichtungsschritt mit einer Schicht porösen, nanoskaligen Silbers überzogen. Hierzu wird unter einer Schutzgasatmosphäre von z.B. Argon bei etwa 10 mbar Arbeitsdruck metallisches Silber verdampft. Dabei wird auf dem Substrat eine Silberschicht (Biozid-Schicht) erzeugt, die aus einzelnen oder untereinander verketteten Silberpartikeln besteht. Die mittlere Primärpartikelgröße der Silberpartikel beträgt etwa 10 bis 20 nm. Die Dicke der Silberschicht (Biozid-Schicht) beträgt etwa 20 nm.

In einem zweiten Beschichtungsschritt wird eine Plasmapolymerschicht mit Hexamethyldisiloxan (HMDSO) als Precursor aufgebracht. Die Plasmapolymerisation wird bei einem Arbeitsdruck von 0,07 mbar mit einem Arbeitsgas aus 95 % O₂ und 5 % HMDSO durchgeführt. Nach 45 Sekunden der so durchgeführten Plasmapolymerisation ist die Silberschicht mit einer 45 nm dicken und stark hydrophilen Plasmapolymerschicht (Transportkontrollschicht) versehen. Die Oberflächenenergie der Beschichtung beträgt dabei 105 mN/m.

Auf diese Weise können insbesondere Medizinprodukte wie Wundauflagen und Katheter mit einem erfindungsgemäßen Schichtmaterial beschichtet werden.

### Beispiel 2: Herstellen eines erfindungsgemäßen Schichtmaterials unter Verwendung einer Haftvermittlungsschicht

Ein mit einem erfindungsgemäßen Schichtmaterial zu versehendes Substrat wird in einem ersten Bearbeitungsschritt mit einem Titandioxidfilm durch Plasmapolymerisation versehen. Als Precursor wird Titantetraisopropyloxid im Gemisch mit Sauerstoff verwendet. Die Polymerisationszeit beträt fünf Minuten. Es entsteht eine 25 nm dicker, gut haftender TiO₂-Film.

In einem zweiten Beschichtungsschritt wird eine dünne metallische Silberschicht im Ultrahochvakuum auf den TiO₂-Film aufgedampft. Während des Aufdampfens beträgt der Prozessdruck 10⁻⁴ mbar. Die Verdampfung wird so durchgeführt, dass eine Silberschicht (Biozid-Schicht) von 10 bis 20 nm Dicke auf dem TiO₂-Film abgeschieden wird.

In einem dritten Beschichtungsschritt wird ein Plasmapolymerfilm (Transportkontrollschicht) auf die Silberschicht aufgetragen. Die Plasmapolymerisation wird wie in Beispiel 1 beschrieben durchgeführt. Es entsteht eine 45 nm dicke und stark hydrophile Plasmapolymerschicht.

Mit dem erfindungsgemäßen Schichtmaterial sind folgende Materialien besonders gut zu versehen: Metalle, insbesondere Titan und (ggf. medizinischer) Edelstahl, Kunststoffe, insbesondere Polyurethan, und Cellulose, insbesondere Wundauflagen und Cellulosevliese.

### Beispiel 3: Auftragen einer Transportkontrollschicht auf einen bioziden Festkörper

Auf einer massiven Kupferschicht wird durch Plasmapolymerisation wie in Beispiel 1 beschrieben ein Plasmapolymerfilm als Transportkontrollschicht abgeschieden. Im Unterschied zu Beispiel 1 wurde die Plasmabeschichtung 450 Sekunden lang durchgeführt. Die so hergestellte Transportkontrollschicht besitzt eine Dicke von 100 nm. Es entsteht ein erfindungsgemäßes Schichtmaterial, wobei die Biozid-Schicht die ursprüngliche, massive Kupferschicht ist.

### Beispiel 4: Untersuchen eines gemäß Beispiel 1 hergestellten erfindungsgemäßen Schichtmaterials

Die Oberfläche der Transportkontrollschicht besitzt laut XPS-Analyse einen Siliciumanteil von 36,6 %, einen Kohlenstoffanteil von 24 % und einen Sauerstoffanteil von 39,4 %. Der Wasserstoffanteil kann mittels XPS-Analyse nicht bestimmt werden. Das Infrarotspektrum des Schichtmaterials zeigt noch einen geringen Anteil an Methylgruppen. Die Transportkontrollschicht ist somit zwar hauptsächlich anorganisch, besitzt jedoch noch eine geringe Konzentration organischer Gruppen.

Das Konzentrationsverhältnis zwischen Silicium und Silber des erfindungsgemäßen Schichtmaterials beträgt laut Energy-Disperse-X-ray-Analyse etwa 10:1. Bezogen auf alle chemischen Elemente (ohne Wasserstoff), die das erfindungsgemäße Schichtmaterial aufbauen, liegt der Silberanteil unter 3 Gew.-%.

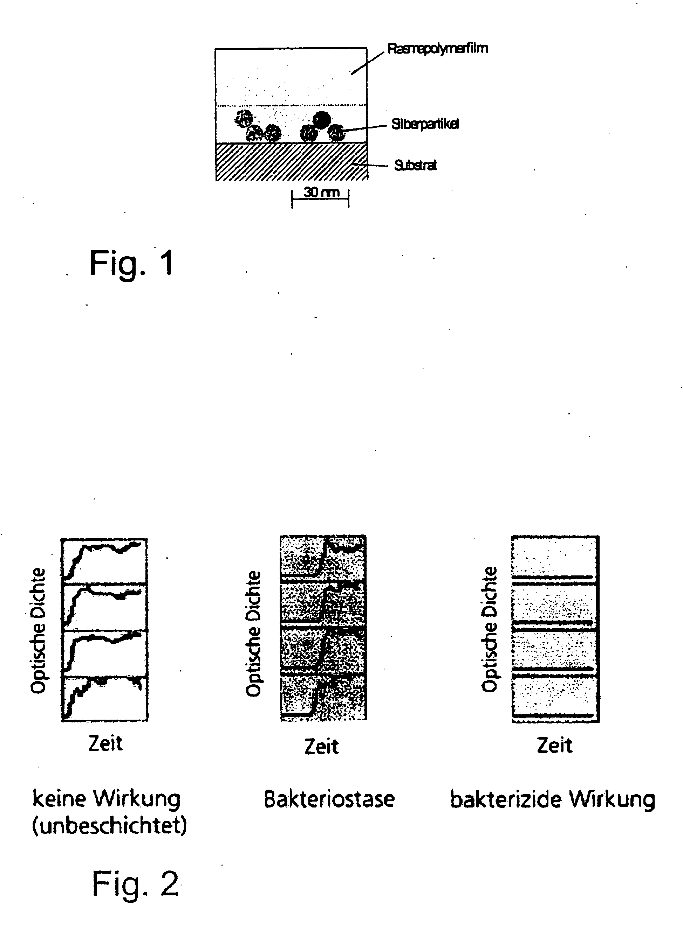

Das Silber ist im erfindungsgemäßen Schichtmaterial nicht homogen verteilt. Figur 1 zeigt schematisch, dass in den vom Substrat abgewandten, äußeren 40 bis 50 nm des Schichtmaterials nur ein sehr geringer Silberanteil vorliegt. Unter dieser äußeren 40 bis 50 nm dicken Schicht (Transportkontrollschicht) befindet sich eine nanoskaliges Silber-enthaltende Schicht von ca. 20 nm Dicke (Biozid-Schicht), die neben Silber auch die übrigen Elemente des Grundmaterials der Transportkontrollschicht enthält. Das nanoskalige Silber ist daher in das Grundmaterial der Transportkontrollschicht als Biozid-Schicht eingebettet.

Figur 2 zeigt einen Nachweis der antimikrobiellen Wirkung einer gemäß Beispiel 1 mit einem erfindungsgemäßen Schichtmaterial versehenen Polyurethan-Oberfläche im Vergleich zu einer unbehandelten Polyurethan-Oberfläche. Die antimikrobielle Wirkung wurde wie in der DE 197 58 598 A1 beschrieben mit *Staphylococcus epidermidis* geprüft. Figur 2 zeigt die Entwicklung der optischen Dichte und damit der Bakterienzahl über einen Zeitraum von 24 Stunden. Die linke Teilfigur zeigt die Entwicklung eines bakteriellen Bewuchses einer unbehandelten Polyurethan-Oberfläche. Die mittlere und die rechte Teilfigur zeigen jeweils die Entwicklungen bakteriellen Bewuchses von mit unterschiedlichen erfindungsgemäßen Schichtmaterialien beschichteten Polyurethan-Oberflächen.

Es ist zu erkennen, dass auf der unbehandelten Polyurethan-Oberfläche innerhalb kürzester Zeit ein bakterielles Wachstum stattfindet, während auf dem erfindungsgemäßen Schichtmaterial innerhalb des dargestellten Zeitraums keine Vermehrung der bakteriellen Zellzahl stattfindet (rechte Teilfigur), oder ein deutlich verzögertes bakterielles Wachstum stattfindet (mittlere Teilfigur). Das erfindungsgemäße Schichtmaterial ist demnach antimikrobiell. Es ist zudem gemäß DIN-ISO10993-5 nicht zytotoxisch (hierzu keine Figur).

### Beispiel 5: Herstellverfahren eines weiteren erfindungsgemäßen Schichtmaterials

Ein festes Substrat, das mit einem erfindungsgemäßen antimikrobiellen und nicht zytotoxischem Schichtmaterial versehen werden soll, wird in einem ersten Beschichtungsschritt mit einer Schicht porösen, nanoskaligen Silbers überzogen. Hierzu wird unter einer Schutzgasatmosphäre von z.B. Argon bei etwa 10 mbar Arbeitsdruck metallisches Silber verdampft. Dabei wird auf dem Substrat eine Silberschicht (Biozid-Schicht) erzeugt, die aus einzelnen oder untereinander verketteten Silberpartikeln besteht. Die mittlere Primärpartikelgröße der Silberpartikel beträgt etwa 10 bis 20 nm. Die Dicke der Silberschicht (Biozid-Schicht) beträgt etwa 20 nm.

In einem zweiten Beschichtungsschritt wird eine Transportkontroll-Plasmapolymerschicht mit Hexamethyldisiloxan (HMDSO) als Precursor aufgebracht. Die Plasma polymerisation wird in einem Reaktor mit einem Volumen von 400 I bei einem Arbeitsdruck von 0.07 mbar, mit einer Plasmaleistung von 2500 W und mit einem Arbeitsgas aus 95 % 02 und 5 % HMDSO durchgeführt. Nach 45 Sekunden der so durchgeführten Plasmapolymerisation ist die Silberschicht mit einer 45 nm dicken Plasmapolymerschicht versehen. Die antimikrobielle Wirkung führt zu einer Verschiebung des Messsignals gemäß DE 197 58 598 A1 um 35 Stunden, so dass das Schichtmaterial praktisch selbststerilisierend ist; jedoch wird die Bakterienadhäsion im Vergleich zum unbeschichteten Polyurethan Substrat nicht gesenkt.

In einem dritten Prozessschritt wird bei einer Leistung von 1500 W, einem Sauerstofffluss von 100 sccm und bei einem Arbeitsdruck von 0.04 mbar zwei Minuten lang eine Sauerstoffaktivierung durchgeführt. Die Oberflächenenergie steigt nach der Sauerstoffaktivierung auf 105 nN/m und die Bakterienadhäsion wird auf ca. 10% des Ausgangswerts gesenkt.

### Beispiel 6: Herstellverfahren eines weiteren erfindungsgemäßen Schichtmaterials

Die Verbindung einer anti-bakteriell wirkenden und einer hämokompatiblen Transportkontrollschicht erfolgt durch die Präparation einer kupferhaltigen Fluorcarbon-Schicht. Die Biozid-Schicht wird durch einen DC-Magnetron-Sputter-Prozess unter Verwendung eines Kupfer-Targets aufgetragen. Bei einem Partialdruck des Ionisationsgases Argon von 5*10-2 mbar entsteht auf dem Substrat eine poröse Cu-Schicht. Die Transportkontrollschicht wird in einem zweiten Arbeitsschritt durch einen Plasmapolymerisationprozess des Precursors Hexafluorethan (C2F6) auf die Biozid-Schicht aufgebracht. Um die Abscheiderate zu erhöhen, wird dem C2F6 Wasserstoff im Verhältnis 3:1 beigemischt. Bei einem Arbeitsdruck von 0.1 mbar entsteht nach einer Prozesszeit von 3 min eine Fluorcarbon-Schicht mit einer Filmdicke von 55 nm und einer Oberflächenenergie von 19 mN/m. In einem letzten Arbeitsschritt wird das Kupfer der Biozid-Schicht durch einen Temperungsschritt bei 50°C in einer sauerstoffhaltigen Atmosphäre zur Kupfer(I)oxid oxidiert. Der Fluor-Anteil in der Transportkontrollschicht entspricht 54.8%, der Kohlenstoffanteil 42.5% und der Sauerstoffanteil 2.7%. Das Fluor selber liegt zu 1/2 als CF2 -, zu 1/3 als CF3- und zu 1/6 als CF-Gruppe vor.

### Beispiel 7: Herstellverfahren eines weiteren erfindungsgemäßen Schichtmaterials

Nach Beispiel 1 wird als Transportkontrollschicht ein Plasmapolymerfilm mit Hexamethyldisiloxan (HMDSO) als Precursor verwendet. Die Oberfläche dieser Schicht kann in einem dritten Arbeitsschritt modifiziert werden. Durch die Abscheidung eines sehr dünnen silikon-ähnlichen Film mit HMDSO als Precursor kann die Oberflächenenergie im Bereich von 105 mN/m bis 22 mN/m beliebig eingestellt werden, ohne die anti-bakteriellen Eigenschaften maßgeblich zu beeinflussen. Über die so modifizierte Oberfläche kann das Anwuchsverhalten von Bakterien aber auch anderer Zellen gesteuert werden.

### Beispiel 8: Herstellverfahren eines weiteren erfindungsgemäßen Schichtmaterials

Wird beim nicht reaktiven DC-Sputtern ein Vanadium-Target eingesetzt, so lassen sich entsprechende poröse Vanadium-Filme als Biozid-Schichten erzeugen. Die Dicke dieser Schicht liegt im Bereich von 50 nm. Als Transportkontrollschicht wird ein Plasmapolymerfilm auf Acrylsäure-Basis mittels Plasmapolymerisation abgeschieden. Nach einer halbstündigen Abscheidung bei einem Acrylsäurefluss von 40 sccm und einem Ar-Fluss von 200 sccm wird ein 50 nm dicker Film aufgebaut, dessen Infrarot-Spektrum dem von Polyacrylsäure entspricht. Die so präparierten Schichten weisen eine hohe und langzeitstabile Oberflächenenergie von ca. 55 mN/m auf.

### Beispiel 9:

Die Transportkontrollschicht muss nicht notwendigerweise durch einen Plasmapolymerisationsprozess hergestellt werden, sondern auch eine reaktiver Mittelfrequenz (MF)-Sputterprozess führt zu einer einsetzbaren Schicht. Das Si-Target wird bei einem Partialdruck des Ionisationsgases Argon von 8*10-4 mbar und bei einem Partialdruck Sauerstoff von 2*10-4 mbar abgesputtert. Diese Schichten sind frei von Kohlenstoff und weisen eine atomare Zusammensetzung von Si:O = 1:2 auf. Neben der Modifizierung der Oberfläche der Transportkontrollschicht lassen sich durch einen Imprägnierungsprozess z.B. Kalziumionen in die Schicht einbringen. Hierzu wird die anti-bakteriell wirkende Schicht für 24 Stunden in eine 0.01 molare Kalziumhydroxidlösung getaucht. Durch einen weiteren Prozessschritt, eine so genannte "Ship-in-a-bottle"-Reaktion, lässt sich das eingelagerte Kalziumhydroxid z.B. in Kalziumchlorid, Kalziumsulfat oder Kalziumcarbonat umwandeln. Zusätzlich zu Kalziumionen können auch über eine nachträgliche Imprägnierung so genannte BMP's (Bone Morphogenetic Proteins) an der Oberfläche der Beschichtung gebunden werden. Die so modifizierten Schichten erlauben ein verbessertes Anwachsen von Knochenzellen.

## Patentansprüche

1. Antimikrobielles und nicht zytotoxisches Schichtmaterial, umfassend
a) eine Biozid-Schicht mit einem bioziden Wirkstoff, und
b) eine die Biozid-Schicht bedeckende Transportkontrollschicht mit einer Dicke und einer Porosität, die eingestellt sind, um den bioziden Wirkstoff aus der Biozid-Schicht durch die Transportkontrollschicht hindurch in einer antimikrobiellen und nicht zytotoxischen Menge abzugeben,
**dadurch gekennzeichnet, dass** die Transportkontrollschicht eine plasmapolymere Schicht und/oder eine aufgesputterte Schicht ist, und wobei die Transportkontrollschicht einen Silizium-Anteil von 20 bis 60 %, einen Kohlenstoffanteil von 10 bis 30 % und einen Sauerstoffanteil von 30 bis 50 % besitzt.

2. Schichtmaterial nach Anspruch 1, **dadurch gekennzeichnet, dass** die Transportkontrollschicht eine Gasdurchlässigkeit für Sauerstoff (O₂) aufweist die im Bereich von 100 bis 1000 (cm³ bar) / (Tag m²), vorzugsweise im Bereich von 500 bis 700 (cm³ bar) / (Tag m²) liegt.

3. Schichtmaterial nach einem der Ansprüche 1 oder 2, wobei der biozide Wirkstoff ausgewählt ist aus der Gruppe bestehend aus Silber, Kupfer und Zink, deren Ionen und deren Metallkomplexe, oder einer Mischung oder Legierung umfassend zwei oder mehr dieser Elemente.

4. Schichtmaterial nach einem der vorherigen Ansprüche , wobei der biozide Wirkstoff ein anorganisches Biozid ist mit einer mittleren Korngröße von 5 bis 100 nm hat.

5. Schichtmaterial nach einem der vorherigen Ansprüche, wobei die Biozid-Schicht ferner umfasst: Gold, Platin, Palladium, Iridium, Zinn, Antimon, deren Ionen, deren Metallkomplexe, oder eine Legierung des bioziden Wirkstoffs mit einem oder mehreren dieser Elemente.

6. Schichtmaterial nach einem der vorherigen Ansprüche, wobei die Transportkontrollschicht ein Grundmaterial besitzt, dass ausgewählt ist aus der Gruppe bestehend aus
a) einem organischen Grundmaterial, insbesondere einem Plasmapolymer, einem Sol-Gel, einem Lack, und einem silikonisierten Grundmaterial, oder
b) einem anorganischen Grundmaterial, insbesondere SiO₂ und SiC, einem Metalloxid, insbesondere TiO₂ und Al₂O₃, und einem nichtbioziden Metall, insbesondere Titan oder medizinischem Edelstahl.

7. Schichtmaterial nach einem der vorherigen Ansprüche, wobei die Biozid-Schicht eine mittlere Dicke von 5 bis 100 nm hat.

8. Schichtmaterial nach einem der vorherigen Ansprüche, wobei die Transportkontrollschicht eine mittlere Dicke von 5 bis 500 nm hat.

9. Medizinprodukt, versehen mit einem antimikrobiellen, nicht zytotoxischen Schichtmaterial nach einem der Ansprüche 1 bis 8.

10. Verfahren zum Herstellen eines antimikrobiellen, nicht zytotoxischen Schichtmaterials nach einem der Ansprüche 1 bis 8 und/oder eines Medizinproduktes nach Anspruch 9, **gekennzeichnet durch** die Schritte:
a) Bereitstellen eines mit einem Biozid versehenen Festkörpers, und
b) Versehen des Festkörpers mit einer Transportkontrollschicht, um den bioziden Wirkstoff aus der Biozid-Schicht **durch** die Transportkontrollschicht hindurch in einer antimikrobiellen und nicht zytotoxischen Menge abzugeben, **durch** Plasmapolymerisation und/oder **durch** Aufsputtern, so dass die Transportkontrollschicht einen Silizium-Anteil von 20 bis 60 %, einen Kohlenstoffanteil von 10 bis 30 % und einen Sauerstoffanteil von 30 bis 50 % besitzt.

## Claims

1. An antimicrobial and non-cytotoxic layered material, comprising:
a) a biocide layer having a biocidal active agent, and
b) a transport control layer covering the biocide layer, having a thickness and porosity adjusted to release an antimicrobial and non-cytotoxic quantity of the biocidal active agent out of the biocide layer and through the transport control layer,
**characterised in that** the transport control layer is a plasma polymer layer and/or a sputter-applied layer and wherein the transport control layer has a silicon content of 20 to 60%, a carbon content of 10 to 30% and an oxygen content of 30 to 50%.

2. A layered material according to claim 1, **characterised in that** the transport control layer has a gas permeability for oxygen (O₂) which is in the range from 100 to 1000 (cm³ bar)/(day m²), preferably in the range from 500 to 700 (cm³ bar) / (day m²).

3. A layered material according to one of claims 1 or 2, wherein the biocidal active agent is selected from the group consisting of silver, copper and zinc, their ions and their metal complexes, or a mixture or alloy comprising two or more of said elements.

4. A layered material according to one of the preceding claims, wherein the biocidal active agent is an inorganic biocide having a mean particle size of 5-100 nm.

5. A layered material according to one of the preceding claims, wherein the biocide layer further comprises: gold, platinum, palladium, iridium, tin, antimony, their ions, their metal complexes, or an alloy of the biocidal active agent with one or more of said elements.

6. A layered material according to one of the preceding claims, wherein the transport control layer has a substrate material that is selected from the group consisting of
a) an organic substrate material, in particular a plasma polymer, a sol-gel, a coating, and a siliconised substrate material, or
b) an inorganic substrate material, in particular SiO₂ and SiC, a metal oxide, in particular TiO₂ and Al₂O₃, and a non-biocidal metal, in particular titanium or medical stainless steel.

7. A layered material according to one of the preceding claims 7, wherein the biocide layer has a mean thickness of 5-100 nm.

8. A layered material according to one of the preceding claims, wherein the transport control layer has a mean thickness of 5-500 nm.

9. A medical product provided with an antimicrobial, non-cytotoxic layered material according to one of claims 1 to 8.

10. A method for producing an antimicrobial, non-cytotoxic layered material according to one of claims 1 to 8 and/or a medical product according to claim 9,
**characterised by** the steps:
a) providing a solid body provided with a biocide, and
b) providing the solid body with a transport control layer, in order to release an antimicrobial and non-cytotoxic quantity of the biocidal active agent out of the biocide layer and through the transport control layer, by plasma polymerisation and/or by sputter application, such that the transport control layer has a silicon content of 20 to 60%, a carbon content of 10 to 30% and an oxygen content of 30 to 50%.

## Revendications

1. Matériau stratifié antimicrobien et non cytotoxique comprenant
a) une couche de biocide ayant un principe actif biocide et
b) une couche de contrôle du transport recouvrant la couche de biocide ayant une épaisseur et une porosité qui sont ajustées pour délivrer le principe actif biocide depuis la couche de biocide au travers de la couche de contrôle du transport en une quantité antimicrobienne et non cytotoxique,
**caractérisé en ce que** la couche de contrôle du transport est une couche polymère à plasma et/ou une couche appliquée par pulvérisation cathodique et où la couche de contrôle du transport possède une proportion de silicium de 20 à 60 %, une proportion de carbone de 10 à 30 % et une proportion d'oxygène de 30 à 50 %.

2. Matériau stratifié selon la revendication 1 **caractérisé en ce que** la couche de contrôle du transport présente une perméabilité aux gaz pour l'oxygène (O₂) qui est située dans le domaine de 100 à 1000 (cm³ bar)/(jour m²), de préférence dans le domaine de 500 à 700 (cm³ bar)/(jour m²).

3. Matériau stratifié selon l'une des revendications 1 ou 2 où le principe actif biocide est choisi dans le groupe consistant en l'argent, le cuivre et le zinc, leurs ions et leurs complexes métalliques, ou un mélange ou alliage comprenant deux ou plusieurs de ces éléments.

4. Matériau stratifié selon l'une des revendications précédentes où le principe actif biocide est un biocide inorganique ayant une granulométrie moyenne de 5 à 100 nm.

5. Matériau stratifié selon l'une des revendications précédentes où la couche de biocide comprend en outre : de l'or, du platine, du palladium, de l'iridium, de l'étain, de l'antimoine, leurs ions, leurs complexes métalliques, ou un alliage du principe actif biocide avec un ou plusieurs de ces éléments.

6. Matériau stratifié selon l'une des revendications précédentes où la couche de contrôle du transport possède un matériau de base qui est choisi dans le groupe consistant en
a) un matériau de base organique, en particulier un polymère à plasma, un sol-gel, une laque et un matériau de base siliconé, ou
b) un matériau de base inorganique, en particulier SiO₂ et SiC, un oxyde métallique, en particulier TiO₂ et Al₂O₃, et un métal non biocide, en particulier le titane ou l'acier spécial médical.

7. Matériau stratifié selon l'une des revendications précédentes où la couche de biocide a une épaisseur moyenne de 5 à 100 nm.

8. Matériau stratifié selon l'une des revendications précédentes où la couche de contrôle du transport a une épaisseur moyenne de 5 à 500 nm.

9. Produit médical muni d'un matériau stratifié antimicrobien non cytotoxique selon l'une des revendications 1 à 8.

10. Procédé pour produire un matériau stratifié antimicrobien non cytotoxique selon l'une des revendications 1 à 8 et/ou un produit médical selon la revendication 9, **caractérisé par** les étapes :
a) préparer un corps solide muni d'un biocide, et
b) munir le corps solide d'une couche de contrôle du transport pour délivrer le principe actif biocide depuis la couche de biocide au travers de la couche de contrôle du transport en une quantité antimicrobienne et non cytotoxique, par polymérisation à plasma et/ou par application par pulvérisation cathodique, de sorte que la couche de contrôle du transport possède une proportion de silicium de 20 à 60 %, une proportion de carbone de 10 à 30 % et une proportion d'oxygène de 30 à 50 %.
